# EUROPEAN PATENT SPECIFICATION

(11) **EP 3 843 670 B1**
(45) Date of publication and mention of the grant of the patent: **11.02.2026**
(21) Application number: 19750010.1
(22) Date of filing: 25.07.2019
(51) Int. Cl.: A61F 5/01, A61F 5/058

(54) **THUMB BRACE AND ITS METHOD OF MANUFACTURE**
DAUMENSCHIENE UND VERFAHREN ZU IHRER HERSTELLUNG
ATTELLE DE POUCE ET SON PROCÉDÉ DE FABRICATION

(30) Priority: 31.08.2018 US 201862725945 P
(43) Date of publication of application: 07.07.2021
(73) Proprietor: DJO France, 64990 Mouguerre (FR)
(72) Inventor: ROUSSEL, Anaïs, 64990 Lahonce (FR); SAGARDIA, Beñat, 64310 Saint-Pée-sur-Nivelle (FR)
(74) Representative: Abel & Imray LLP
(86) International application number: PCT/US2019/043441
(87) International publication number: WO 2020/046501

(56) References cited:
- EP-A1- 1 905 399
- WO-A1-2013/160478
- NL-B1- 2 016 047
- US-A- 4 665 907
- US-A1- 2017 348 191

## Description

### BACKGROUND

### Field of the Disclosure

The present disclosure relates to braces for immobilizing an appendage of a user. Specifically, the apparatuses and methods described herein provide a thumb brace for at least partially immobilizing a thumb of the user.

### Description of the Related Technology

Thumb braces have been used in the past to partially or fully immobilize a thumb of a hand of a user in treating or avoiding any number of injuries to the thumb, hand or particular joints of the same. However, conventional thumb braces may be bulky, uncomfortable or extend across a larger portion of the hand than desirable if the user wishes to retain as much use of the affected hand as possible.

Accordingly, there is a need for solutions that provide a less bulky, more comfortable and less intrusive brace that sufficiently supports, immobilizes and/or protects a thumb, hand, or particular joints of the same while maintaining a greater use of the affected hand.

US 2017/348191 A1, published 7 December 2017, relates to a headache-relieving device including a device body having a first side, a second side and a connecting portion connecting the second side to the first side. A hand space is formed between the first side and the second side allowing a user to insert his or her hand. A pressing element is provided on the second side. The pressing element is configured to apply pressure against a pressure point of a user's hand positioned in the hand space.

EP 1905399 A1, published 2 April 2008, relates to an orthopedic device for immobilizing a thumb, including a main body having a wrist end and a thumb end. A wrist strap is located on the wrist end of the main body for fastening the wrist end of the main body to the wrist of a user, and a thumb strap is located on the thumb end of the main body for fastening the thumb end of the main body to the wrist of a user. The thumb strap is adapted to encircle a joint of a user's thumb. When the wrist and thumb straps are fastened, the main body substantially immobilizes the thumb. US 4665907 A, published 19 May 1987, relates to an apparatus for attaching to the thumb or finger to inhibit thumb sucking. A bracelet fits around the wrist, and a primary ring attaches to the bracelet. The primary ring is supported by two or more tabs extending outwardly from the ring to the bracelet, and cross-tabs extend between the outward tabs to prevent unwanted withdrawal of the thumb from the ring. A separate booster ring is optionally attached to the primary thumb-encircling ring, providing additional hindrance to thumb sucking.

### SUMMARY

The invention is defined by the accompanying claims.

### BRIEF DESCRIPTION OF THE DRAWINGS

**FIG. 1** illustrates a palm-side view of at least a portion of a thumb brace for at least partially immobilizing a thumb of a user, according to some embodiments;
**FIG. 2** illustrates a back-side view of at least a portion of the thumb brace of **FIG. 1****,** according to some embodiments;
**FIG. 3** illustrates a view of at least a portion of the thumb brace of **FIGs. 1** and **2** along a side of the hand defined by a thumb metacarpal of the hand, according to some embodiments;
**FIG. 4** illustrates a flowchart of a method for using a thumb brace for at least partially immobilizing a thumb of a user, according to some embodiments.
**FIG. 5** illustrates a flowchart of a method for manufacturing a thumb brace for at least partially immobilizing a thumb of a user, according to some embodiments.

### DETAILED DESCRIPTION

Various aspects of the novel braces and associated methods are described more fully hereinafter with reference to the accompanying drawings. However, the disclosure may contemplate many different forms of such braces and associated methods and should not be construed as limited to any specific structure or function presented throughout this disclosure. Rather, these aspects are provided so that this disclosure will be thorough and complete, and will fully convey the scope of the disclosure to those skilled in the art. Based on the teachings herein one skilled in the art should appreciate that the scope of the disclosure is intended to cover any aspect of the novel braces and methods disclosed herein, whether implemented independently of or combined with any other aspect of the disclosure. For example, a brace may be implemented, or a method may be practiced using any number of the aspects set forth herein. In addition, the scope of the disclosure is intended to cover such a brace or method which is practiced using other structure, functionality, or structure and functionality in addition to or other than the various aspects of the disclosure set forth herein. It should be understood that any aspect disclosed herein may be embodied by one or more elements of a claim.

Although particular aspects are described herein, many variations and permutations of these aspects fall within the scope of the disclosure. Although some benefits and advantages of the preferred aspects are mentioned, the scope of the disclosure is not intended to be limited to particular benefits, uses, or objectives. Rather, aspects of the disclosure are intended to be broadly applicable to different applications, some of which are illustrated by way of example in the figures and in the following description of the preferred aspects. The detailed description and drawings are merely illustrative of the disclosure rather than limiting, the scope of the disclosure being defined by the appended claims and equivalents thereof.

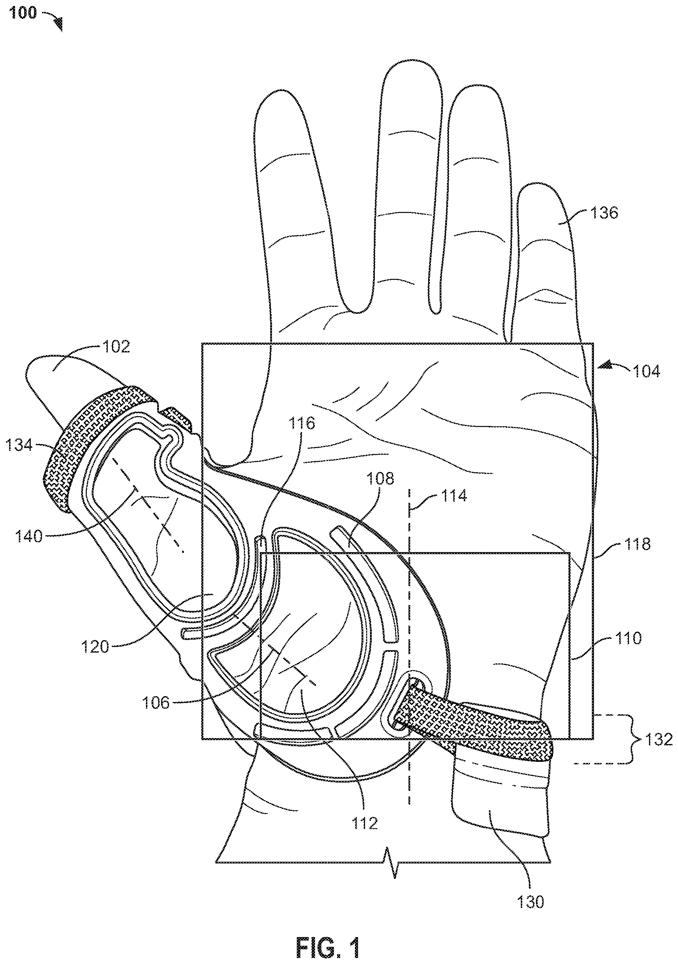

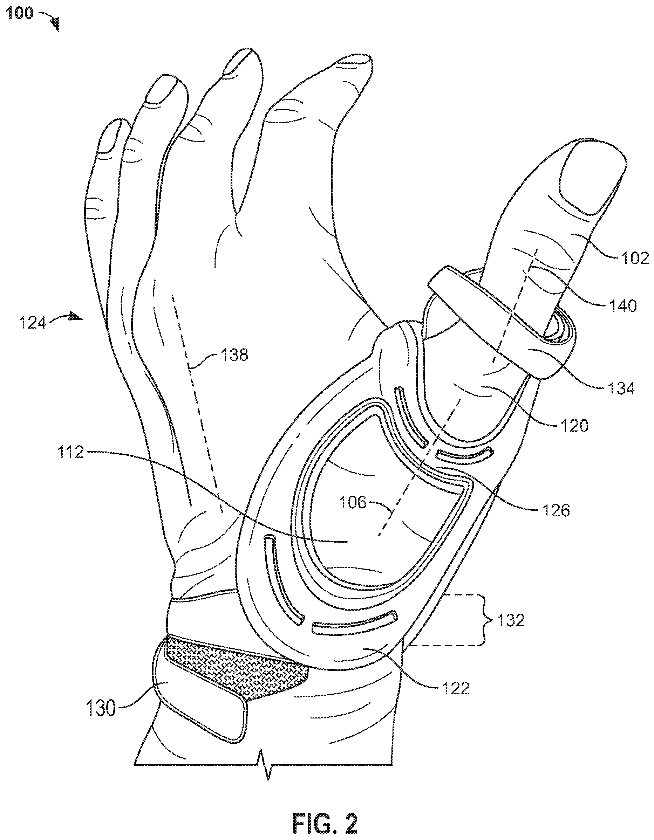

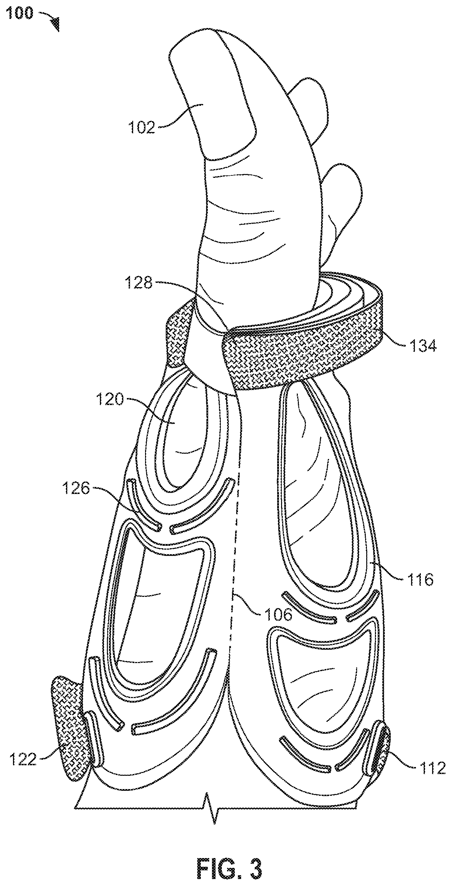

Several aspects of a thumb brace for at least partially immobilizing a thumb of a user will now be described in connection with **FIGs. 1-3** together below. **FIG. 1** illustrates a palm-side view of at least a portion of a thumb brace 100 for at least partially immobilizing a thumb 102 of a user, according to some embodiments. **FIG. 2** illustrates a back-side view of at least a portion of thumb brace 100 of **FIG. 1****,** according to some embodiments. **FIG. 3** illustrates a view of at least a portion of thumb brace 100 of **FIGs. 1** and **2** along a side of a hand 104 defined by a thumb metacarpal 106 of hand 104, according to some embodiments.

Thumb brace 100 comprises a first curved support element 108 configured to be disposed on a heel 110 of hand 104 along an intercarpal articulation 112 of thumb 102 and in a palmar gutter 114 of hand 104. First curved support element 108 secures thumb brace 100 within palmar gutter 114 and substantially surrounds at least a proximal portion of a thumb metacarpal 106 on a palm 118 and/or heel 110 of hand 104. Disposing first curved support element 108 along intercarpal articulation 112 of thumb 102 and in palmar gutter 114 of hand 104 ensures first curved support element 108 does not move substantially with respect to hand 104 once secured.

Thumb brace 100 comprises a second curved support element 116 coupled to first curved support element 108 and configured to be disposed on palm 118 of hand 104 along a metacarpophalangeal joint 120 of thumb 102. Second curved support element 116 substantially surrounds at least a proximal portion of metacarpophalangeal joint 120 of thumb 102 on palm 118 of hand 104 between a thumb phalange 140 and thumb metacarpal 106. Disposing second curved support element 116 on palm 118 of hand 104 along a metacarpophalangeal joint 120 of thumb 102 ensures second curved support element 116 secures thumb phalange 140 at substantially it's most distal extent, thereby providing a greater amount of leverage in preventing thumb 102 from substantially moving with respect to the rest of hand 104 once secured.

As seen best with reference to Figure 2, thumb brace 100 comprises a third curved support element 122 configured to be disposed on a backside 124 of hand 104 along intercarpal articulation 112 of thumb 102. Third curved support element 122 substantially surrounds a proximal portion of intercarpal articulation 112 of thumb 102 along a backside and/or side of a wrist 132 of hand 104. Disposing third curved support element 122 to substantially surround the proximal portion of intercarpal articulation 112 of thumb 102 along the backside and/or side of wrist 132 of hand 104 ensures third curved support element 122 secures thumb phalange 140 at substantially it's most proximal extent.

Thumb brace 100 comprises a fourth curved support element 126 coupled to third curved support element 122 and configured to be disposed on backside 124 of hand 104 along metacarpophalangeal joint 120 of thumb 102. Fourth curved support element 126 substantially surrounds at least a proximal portion of metacarpophalangeal joint 120 of thumb 102 on backside 124 of hand 104 between thumb phalange 140 and thumb metacarpal 106. Disposing fourth curved support element 126 on backside 124 of hand 104 along metacarpophalangeal joint 120 of thumb 102 ensures fourth curved support element 126 secures thumb phalange 140 at substantially it's most distal extent, thereby providing a greater amount of leverage in preventing thumb 102 from substantially moving with respect to the rest of hand 104 once secured.

In some embodiments, first curved support element 108, second curved support element 116, third curved support element 122, and fourth curved support element 126 may each have a concave curvature that corresponds to a respective local curvature of a portion of hand 104 on which that element is configured to contact, thereby allowing each element 108, 116, 122, 126 to seat securely against respective portions of hand 104 and/or thumb 102.

In some embodiments, one or more of first curved support element 108, second curved support element 116, third curved support element 122, and fourth curved support element 126 may comprise respective strips of an elastomeric material or a fabric reinforced, internally or externally, with a semi-rigid material, such as metal, for example, aluminum. However, the present disclosure is not so limited and first curved support element 108, second curved support element 116, third curved support element 122, and fourth curved support element 126 may comprise any suitable material and may be reinforced internally, externally, or both with any suitable semi-rigid or rigid material, such as but not limited to plastic, ceramic, or acrylic materials.

Thumb brace 100 comprises a joining element 128 coupling first curved support element 108, second curved support element 116, third curved support element 122, and fourth curved support element 126 along a side of hand 104 defined by thumb metacarpal 106 of hand 104. Joining element 128 allows first curved support element 108 and second curved support element 116 to align properly on palm 118 and thumb 102 of the hand, and third curved support element 122 and fourth curved support element 126 to align properly on backside 124 and thumb 102 of hand 104. Moreover, joining element 128 ensures that none of first curved support element 108, second curved support element 116, third curved support element 122, and fourth curved support element 126 wrap around thumb 102 from palm 118 to backside 124 of hand 104. As shown in **FIGs. 1-3****,** first curved support element 108 does not extend laterally toward a digitus minimus manus (hereinafter, "little finger") 136 of hand 104 substantially beyond palmar gutter 114. Similarly, third curved support element 122 does not extend laterally toward little finger 136 of hand 104 substantially beyond a midline 138 of backside 124 of hand 104. Accordingly, thumb brace 100 does not unnecessarily contact portions of hand 104 that would prevent, hinder or complicate use of hand 104 in ways that do not require substantial movement of thumb 102 with respect to hand 104. In some embodiments, two or more of joining element 128, first curved support element 108, second curved support element 116, third curved support element 122, and fourth curved support element 126 may be formed as a single or unitary piece.

Thumb brace 100 comprises a second strap 130 coupled to first curved support element 108 and configured to wrap around wrist 132 of hand 104 and couple to third curved support element 122. Second strap 130 secures thumb brace 100, and specifically first and third curved support elements 108, 122 and joining element 128 against respective portions of hand 104. Thumb brace 100 comprises a first strap 134 coupled to joining element 128 and configured to wrap around thumb 102 and couple to at least one of second and fourth curved support elements 112, 126. First strap 134 secures thumb brace 100, and specifically joining element 128 and so second and fourth curved support elements 116, 126 against respective portions of hand 104 and/or thumb 102. By disposing first strap 134 such that it secures to and/or around thumb 102, thumb 102 is secured at a position distal of metacarpophalangeal joint 120 of thumb 102, thereby ensuring substantially immobility of thumb 102 with respect to hand 102.

Second strap 130 and first strap 134 may comprise fabric, leather, plastic, or any other suitable material having sufficient strength and durability to secure portions of thumb brace 100 as described above. In some embodiments, second strap 130 and first strap 134 may comprise hook and loop fasteners, buttons, snaps, pins or any other suitable type of fastener.

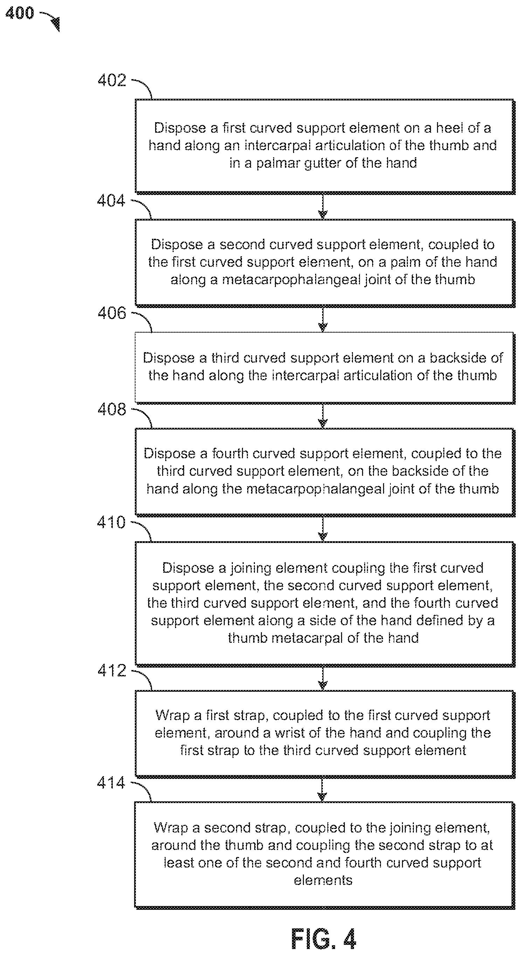

**FIG. 4** illustrates a flowchart 400 of a method for using a thumb brace for at least partially immobilizing a thumb of a user, according to some embodiments. Flowchart 400 will now be described in connection with thumb brace 100 as previously described in connection with **FIGs. 1-3** above.

Block 402 includes disposing a first curved support element on a heel of a hand along an intercarpal articulation of a thumb and in a palmar gutter of the hand. For example, as previously described in connection with **FIGs. 1-3****,** first curved support element 108 may be configured to be disposed on heel 110 of hand 104 along intercarpal articulation 112 of thumb 102 and in palmar gutter 114 of hand 104.

Block 404 includes disposing a second curved support element, coupled to the first curved support element, on a palm of the hand along a metacarpophalangeal joint of the thumb. For example, as previously described in connection with **FIGs. 1-3****,** second curved support element 116 may be coupled to first curved support element 108 and may be configured to be disposed on palm 118 of hand 104 along metacarpophalangeal joint 120 of thumb 102.

Block 406 includes disposing a third curved support element on a backside of the hand along the intercarpal articulation of the thumb. For example, as previously described in connection with **FIGs. 1-3****,** third curved support element 122 may be configured to be disposed on backside 124 of hand 104 along intercarpal articulation 112 of thumb 102.

Block 408 includes disposing a fourth curved support element, coupled to the third curved support element, on the backside of the hand along the metacarpophalangeal joint of the thumb. For example, as previously described in connection with **FIGs. 1-3****,** fourth curved support element 126 may be coupled to third curved support element 122 and configured to be disposed on backside 124 of hand 104 along metacarpophalangeal joint 120 of thumb 102.

Block 410 includes disposing a joining element coupling the first curved support element, the second curved support element, the third curved support element, and the fourth curved support element along a side of the hand defined by a thumb metacarpal of the hand. For example, as previously described in connection with **FIGs. 1-3****,** joining element 128 may be disposed to couple first curved support element 108, second curved support element 116, third curved support element 122, and fourth curved support element 126 along a side of hand 104 defined by thumb metacarpal 106 of hand 104.

Block 412 includes wrapping a second strap, coupled to the first curved support element, around a wrist of the hand and coupling the first strap to the third curved support element. For example, as previously described in connection with **FIGs. 1-3****,** second strap 130 may be coupled to first curved support element 108 and second strap 130 may be configured to wrap around wrist 132 of hand 104 and couple to third curved support element 122.

Block 414 includes wrapping a first strap, coupled to the joining element, around the thumb and coupling the first strap to at least one of the second and fourth curved support elements. For example, as previously described in connection with **FIGs. 1-3****,** first strap 134 may be coupled to joining element 128 and first strap 134 may be configured to wrap around thumb 102 and may be coupled to at least one of second and fourth curved support elements 116, 126.

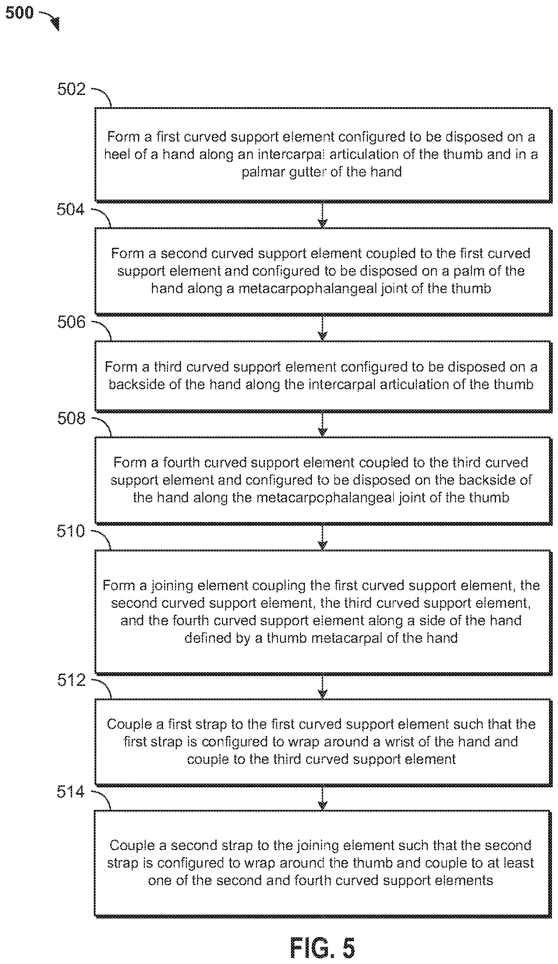

**FIG. 5** illustrates a flowchart 500 of a method for manufacturing a thumb brace for at least partially immobilizing a thumb of a user, according to some embodiments. Flowchart 500 will now be described in connection with thumb brace 100 as previously described in connection with **FIGs. 1-3** above.

Block 502 includes forming a first curved support element configured to be disposed on a heel of a hand along an intercarpal articulation of the thumb and in a palmar gutter of the hand. For example, as previously described in connection with **FIGs. 1-3****,** upon proper formation, first curved support element 108 may be configured to be disposed on heel 110 of hand 104 along intercarpal articulation 112 of thumb 102 and in palmar gutter 114 of hand 104.

Block 504 includes forming a second curved support element coupled to the first curved support element and configured to be disposed on a palm of the hand along a metacarpophalangeal joint of the thumb. For example, as previously described in connection with **FIGs. 1-3****,** upon proper formation, second curved support element 116 may be coupled to first curved support element 108 and may be configured to be disposed on palm 118 of hand 104 along metacarpophalangeal joint 120 of thumb 102.

Block 506 includes forming a third curved support element configured to be disposed on a backside of the hand along the intercarpal articulation of the thumb. For example, as previously described in connection with **FIGs. 1-3****,** upon proper formation, third curved support element 122 may be configured to be disposed on backside 124 of hand 104 along intercarpal articulation 112 of thumb 102.

Block 508 includes forming a fourth curved support element coupled to the third curved support element and configured to be disposed on the backside of the hand along the metacarpophalangeal joint of the thumb. For example, as previously described in connection with **FIGs. 1-3****,** upon proper formation, fourth curved support element 126 may be coupled to third curved support element 122 and may be configured to be disposed on backside 124 of hand 104 along metacarpophalangeal joint 120 of thumb 102.

Block 510 includes forming a joining element coupling the first curved support element, the second curved support element, the third curved support element, and the fourth curved support element along a side of the hand defined by a thumb metacarpal of the hand. For example, as previously described in connection with **FIGs. 1-3****,** upon proper formation, joining element 128 may couple first curved support element 108, second curved support element 116, third curved support element 122, and fourth curved support element 126 along a side of hand 104 defined by thumb metacarpal 106 of hand 104.

Block 512 includes coupling a second strap to the first curved support element such that the second strap is configured to wrap around a wrist of the hand and couple to the third curved support element. For example, as previously described in connection with **FIGs. 1-3****,** upon proper coupling, second strap 130 may be coupled to first curved support element 108 and second strap 130 may be configured to wrap around wrist 132 of hand 104 and couple to third curved support element 122.

Block 514 includes coupling a first strap to the joining element such that the first strap is configured to wrap around the thumb and couple to at least one of the second and fourth curved support elements. For example, as previously described in connection with **FIGs. 1-3****,** upon proper coupling, first strap 134 may be coupled to joining element 128 and first strap 134 may be configured to wrap around thumb 102 and may be coupled to at least one of second and fourth curved support elements 116, 126.

Various modifications to the implementations described in this disclosure can be readily apparent to those skilled in the art, and any generic principles defined herein can be applied to other implementations without departing from the spirit or scope of this disclosure. Thus, the disclosure is not intended to be limited to the implementations shown herein but is to be accorded the widest scope consistent with the claims, the principles and the novel features disclosed herein. The word "exemplary" is used exclusively herein to mean "serving as an example, instance, or illustration." Any implementation described herein as "exemplary" is not necessarily to be construed as preferred or advantageous over other implementations.

Certain features that may be described in the context of separate implementations also can be implemented in combination in a single implementation. Conversely, various features that are described in the context of a single implementation also can be implemented in multiple implementations separately or in any suitable sub-combination. Moreover, although features can be described above as acting in certain combinations and even initially claimed as such, one or more features from a claimed combination can in some cases be excised from the combination, and the claimed combination can be directed to a sub-combination or variation of a sub-combination.

In addition, the methods disclosed herein comprise one or more steps or actions for achieving the described method. The method steps and/or actions may be interchanged with one another without departing from the scope of the claims. In other words, unless a specific order of steps or actions is specified, the order and/or use of specific steps and/or actions may be modified without departing from the scope of the claims.

While the foregoing is directed to aspects of the present disclosure, other and further aspects of the disclosure may be devised without departing from the basic scope thereof, and the scope thereof is determined by the claims that follow.

## Claims

1. A thumb brace (100) for at least partially immobilizing a thumb (102) of a user, comprising:
a first curved support strip (108) configured to be disposed along an intercarpal articulation (112) of the thumb and in a palmar gutter (114) on a heel (110) of a hand (104);
a second curved support strip (116) coupled to the first curved support strip and configured to extend along a metacarpophalangeal joint (120) of the thumb on a palm (118) of the hand and, thereby, expose a portion of the palm between the first and second curved support strips;
a third curved support strip (122) configured to be disposed along the intercarpal articulation of the thumb on a backside (124) of the hand;
a fourth curved support strip (126) coupled to the third curved support strip and configured to extend along the metacarpophalangeal joint of the thumb on the backside of the hand and, thereby, expose a portion of the backside of the hand between the third and fourth curved support strips;
a joining element (128) coupling the first curved support strip, the second curved support strip, the third curved support strip, and the fourth curved support strip along a side of the hand defined by a thumb metacarpal (106) of the hand; and
a first strap (134) coupled to the joining element, the first strap configured to wrap around the thumb and coupled to at least one of the second and fourth curved support strips,
**characterized in that**:
one or more of the first curved support strip, the second curved support strip, the third curved support strip, and the fourth curved support strip, has a concave curvature, and
the concave curvature corresponds to a respective local curvature of a portion of the hand on which at least one of the one or more of the first, second, third, and fourth curved support strips is configured to contact.

2. The thumb brace of claim 1, further comprising a second strap (130) coupled to the first curved support strip, the first strap configured to wrap around a wrist (132) of the hand and couple to the third curved support strip.

3. The thumb brace of claim 1, wherein the first curved support strip does not extend laterally toward a little finger (136) of the hand substantially beyond the palmar gutter.

4. The thumb brace of claim 1, wherein the third curved support strip does not extend laterally toward a little finger (136) of the hand substantially beyond a midline (138) of the hand.

5. The thumb brace of claim 1, wherein the first curved support strip substantially surrounds at least a proximal portion of a thumb metacarpal (106) on the palm and/or the heel of the hand.

6. The thumb brace of claim 1, wherein the second curved support strip 116 substantially surrounds at least a proximal portion of the metacarpophalangeal joint of the thumb on the palm of the hand between a thumb phalange (140) and a thumb metacarpal (106).

7. The thumb brace of claim 1, wherein the third curved support strip substantially surrounds a proximal portion of the intercarpal articulation of the thumb along a backside and/or a side of a wrist (132) of the hand.

8. The thumb brace of claim 1, wherein the fourth curved support strip substantially surrounds at least a proximal portion of the metacarpophalangeal joint of the thumb on the backside of the hand between a thumb phalange (140) and a thumb metacarpal (106).

9. The thumb brace of claim 1, wherein the first, second, third and fourth curved support strips comprise strips of an elastomeric material or a fabric, the elastomeric material or the fabric reinforced with a semi-rigid material.

10. The thumb brace of claim 1, wherein none of the first, second, third or fourth curved support strips wrap around the thumb from the palm to the backside of the hand.

11. A method of manufacturing a thumb brace (100) for at least partially immobilizing a thumb (102) of a user, the method comprising:
forming a first curved support strip (108) configured to be disposed along an intercarpal articulation (112) of the thumb and in a palmar gutter (114) on a heel (110) of a hand (104);
forming a second curved support strip (116) coupled to the first curved support strip and configured extend along a metacarpophalangeal joint (120) of the thumb on a palm (118) of the hand and, thereby, expose a portion of the palm between the first and second curved support strips;
forming a third curved support strip (122) configured to be disposed along the intercarpal articulation of the thumb on a backside (124) of the hand;
forming a fourth curved support strip (126) coupled to the third curved support strip and configured to extend along the metacarpophalangeal joint of the thumb on the backside of the hand and, thereby expose a portion of the backside of the between the third and fourth curved support strips;
forming a joining element (128) coupling the first curved support strip, the second curved support strip, the third curved support strip, and the fourth curved support strip along a side of the hand defined by a thumb metacarpal (106) of the hand; and
coupling a first strap (134) to the joining element such that the first strap is configured to wrap around the thumb and couple to at least one of the second and fourth curved support strips,
**characterized in that**:
at least one of the first curved support strip, the second curved support strip, the third curved support strip, and the fourth curved support strip, has a concave curvature, and
the concave curvature is formed to correspond to a respective local curvature of a portion of the hand on which at least one of the first, second, third, and fourth curved support strips is configured to contact.

12. The method of claim 11, further comprising:
coupling a second strap (130) to the first curved support strip such that the first strap is configured to wrap around a wrist (132) of the hand and couple to the third curved support strip.

## Patentansprüche

1. Daumenschiene (100) zum zumindest teilweisen Ruhigstellen eines Daumens (102) eines Benutzers, mit
einem ersten gekrümmten Stützstreifen (108), der derart konfiguriert ist, dass er entlang eines Interkarpalgelenks (112) des Daumens und in einer Handflächenrinne (114) in einem Handballen (110) einer Hand (104) angeordnet werden kann,
einem zweiten gekrümmten Stützstreifen (116), der mit dem ersten gekrümmten Stützstreifen verbunden ist und derart konfiguriert ist, dass er sich entlang eines Metakarpophalangealgelenks (120) des Daumens auf einer Handfläche (118) der Hand erstreckt und dadurch einen Teil der Handfläche zwischen dem ersten und dem zweiten gekrümmten Stützstreifen freilegt,
einem dritten gekrümmten Stützstreifen (122), der derart konfiguriert ist, dass er entlang des Interkarpalgelenks des Daumens auf einer Rückseite (124) der Hand angeordnet werden kann,
einem vierten gekrümmten Stützstreifen (126), der mit dem dritten gekrümmten Stützstreifen verbunden ist und derart konfiguriert ist, dass er sich entlang des Metakarpophalangealgelenks des Daumens auf der Rückseite der Hand erstreckt und dadurch einen Teil der Rückseite der Hand zwischen dem dritten und dem vierten gekrümmten Stützstreifen freilegt,
einem Verbindungselement (128), das den ersten gekrümmten Stützstreifen, den zweiten gekrümmten Stützstreifen, den dritten gekrümmten Stützstreifen und den vierten gekrümmten Stützstreifen entlang einer Seite der Hand verbindet, die durch einen Daumen-Mittelhandknochen (106) der Hand definiert ist, und
einem ersten Band (134), das mit dem Verbindungselement verbunden ist, bei der das erste Band derart konfiguriert ist, dass es um den Daumen gewickelt wird und mit mindestens einem von dem zweiten und dem vierten Stützstreifen verbunden ist,
**dadurch gekennzeichnet, dass**
mindestens einer von dem ersten gekrümmten Stützstreifen, dem zweiten gekrümmten Stützstreifens, dem dritten gekrümmten Stützstreifen und dem vierten gekrümmten Stützstreifen eine konkave Krümmung aufweist, und
die konkave Krümmung einer jeweiligen lokalen Krümmung eines Teils der Hand entspricht, auf dem zumindest einer von dem mindestens einem von dem ersten, zweiten, dritten und vierten gekrümmtem Stützstreifen so konfiguriert ist, dass er aufliegt.

2. Daumenschiene nach Anspruch 1, ferner mit einem zweiten Band (130), das mit dem ersten gekrümmten Stützstreifen verbunden ist, bei der das erste Band dazu konfiguriert ist, dass es um ein Handgelenk (132) der Hand gewickelt wird und mit dem dritten gekrümmten Stützstreifen verbindet.

3. Daumenschiene nach Anspruch 1, bei der sich der erste gekrümmte Stützstreifen nicht lateral in Richtung eines kleinen Fingers (136) der Hand im Wesentlichen über die Handflächenrinne erstreckt.

4. Daumenschiene nach Anspruch 1, bei der sich der dritte gekrümmte Stützstreifen nicht lateral in Richtung eines kleinen Fingers (136) der Hand im Wesentlichen über eine Mittellinie (138) der Hand erstreckt.

5. Daumenschiene nach Anspruch 1, bei der der erste gekrümmte Stützstreifen im Wesentlichen zumindest einen proximalen Bereich eines Metakarpophalangealgelenks (106) des Daumens auf der Handfläche und/oder dem Handballen umgibt.

6. Daumenschiene nach Anspruch 1, bei der der zweite gekrümmte Stützstreifen (116) im Wesentlichen zumindest einen proximalen Bereich des Metakarpophalangealgelenks des Daumens auf der Handfläche zwischen einem Daumenglied (140) und einem Daumen-Mittelhandknochen (106) umgibt.

7. Daumenschiene nach Anspruch 1, bei der der dritte gekrümmte Stützstreifen im Wesentlichen einen proximalen Bereich des Interkarpalgelenks des Daumens entlang einer Rückseite und/oder einer Seite eines Handgelenks (132) der Hand umgibt.

8. Daumenschiene nach Anspruch 1, bei der der vierte gekrümmte Stützstreifen im Wesentlichen zumindest einen proximalen Bereich des Metakarpophalangealgelenks des Daumens auf der Rückseite der Hand zwischen einem Daumenglied (140) und einem Daumen-Mittelhandknochen (106) umgibt.

9. Daumenschiene nach Anspruch 1, bei der der erste, zweite, dritte und vierte gekrümmte Stützstreifen aus einem Elastomermaterial oder einem Gewebe hergestellt sind, bei der das Elastomermaterial oder das Gewebe mit einem halbstarren Material verstärkt ist.

10. Daumenschiene nach Anspruch 1, bei der keiner von dem ersten, dem zweiten, dem dritten oder dem vierten gekrümmten Stützstreifen um den Daumen von der Handfläche zu der Rückseite der Hand gewickelt ist.

11. Verfahren zum Herstellen einer Daumenschiene (100) zum zumindest teilweisen Ruhigstellen eines Daumens (102) eines Benutzers, umfassend:
Ausbilden eines ersten gekrümmten Stützstreifens (108), der derart konfiguriert ist, dass er entlang eines Interkarpalgelenks (112) des Daumens und in einer Handflächenrinne (114) an einem Handballen (110) einer Hand (104) angeordnet werden kann,
Ausbilden eines zweiten gekrümmten Stützstreifens (116), der mit dem ersten gekrümmten Stützstreifen verbunden ist und derart konfiguriert ist, dass er sich entlang eines Metakarpophalangealgelenks (120) des Daumens auf einer Handfläche (118) der Hand erstreckt und dadurch einen Teil der Handfläche zwischen dem ersten und dem zweiten gekrümmten Stützstreifen freilegt,
Ausbilden eines dritten gekrümmten Stützstreifens (122), der derart konfiguriert ist, dass er entlang des Interkarpalgelenks des Daumens auf einer Rückseite (124) der Hand angeordnet werden kann,
Ausbilden eines vierten gekrümmten Stützstreifens (126), der mit dem dritten gekrümmten Stützstreifen verbunden ist und derart konfiguriert ist, dass er sich entlang des Metakarpophalangealgelenks des Daumens auf der Rückseite der Hand erstreckt und dadurch einen Teil der Rückseite zwischen dem dritten und dem vierten gekrümmten Stützstreifen freilegt,
Ausbilden eines Verbindungselements (128), das den ersten gekrümmten Stützstreifen, den zweiten gekrümmten Stützstreifen, den dritten gekrümmten Stützstreifen und den vierten gekrümmten Stützstreifen entlang einer Seite der Hand verbindet, die durch einen Daumen-Mittelhandknochen (106) der Hand definiert ist, und
Verbinden eines ersten Bandes (134) mit dem Verbindungselement, so dass das erste Band dazu konfiguriert ist, dass es um den Daumen gewickelt wird und mit mindestens einem von dem zweiten und vierten gekrümmten Stützstreifen verbunden ist,
**dadurch gekennzeichnet, dass**
mindestens einer von dem ersten gekrümmten Stützstreifen, dem zweiten gekrümmten Stützstreifen, dem dritten gekrümmten Stützstreifen und dem vierten gekrümmten Stützstreifen eine konkave Krümmung aufweist, und
die konkave Krümmung derart ausgebildet ist, dass sie einer jeweiligen lokalen Krümmung eines Teils der Hand entspricht, auf dem mindestens einer von dem ersten, zweiten, dritten und vierten gekrümmten Stützstreifen so konfiguriert ist, dass er aufliegt.

12. Verfahren nach Anspruch 11, ferner umfassend:
Verbinden eines zweiten Bandes (130) an den ersten gekrümmten Stützstreifen, so dass das erste Band dazu konfiguriert ist, dass es um ein Handgelenk (132) der Hand gewickelt wird und mit dem dritten gekrümmten Stützstreifen verbindet.

## Revendications

1. Attelle de pouce (100) pour immobiliser au moins partiellement le pouce (102) d'un utilisateur, comprenant :
une première bande de support incurvée (108) configurée pour être disposée sur un talon (110) d'une main (104) le long d'une articulation intercarpienne (112) du pouce et dans la gouttière palmaire (114) ;
une deuxième bande de support incurvée (116) couplée à la première bande de support incurvée et configurée pour s'étendre le long d'une articulation métacarpo-phalangienne (120) du pouce sur une paume (118) de la main et, ainsi, exposer une partie de la paume entre les première et deuxième bandes de support incurvées ;
une troisième bande de support incurvée (122) configurée pour être disposée le long de l'articulation intercarpienne du pouce sur une face arrière (124) de la main ;
une quatrième bande de support incurvée (126) couplée à la troisième bande de support incurvée et configurée pour s'étendre le long de l'articulation métacarpo-phalangienne du pouce sur la face arrière de la main et, ainsi, exposer une partie de la face arrière de la main entre les troisième et quatrième bandes de support incurvées ;
un élément de jonction (128) couplant la première bande de support incurvée, la deuxième bande de support incurvée, la troisième bande de support incurvée et la quatrième bande de support incurvée le long d'un côté de la main défini par un métacarpien de pouce (106) de la main ; et
une première sangle (134) couplée à l'élément de jonction, la première sangle étant configurée pour s'enrouler autour du pouce et couplée à au moins une des deuxième et quatrième bandes de support incurvées,
**caractérisée en ce que** :
une ou plusieurs de la première bande de support incurvée, de la deuxième bande de support incurvée, de la troisième bande de support incurvée et de la quatrième bande de support incurvée, présentent une courbure concave, et
la courbure concave correspond à une courbure locale respective d'une partie de la main sur laquelle au moins une des une ou plusieurs des première, deuxième, troisième et quatrième bandes de support incurvées est configurée pour venir en contact.

2. Attelle de pouce selon la revendication 1, comprenant en outre une seconde sangle (130) couplée à la première bande de support incurvée, la première sangle étant configurée pour s'enrouler autour d'un poignet (132) de la main et se coupler à la troisième bande de support incurvée.

3. Attelle de pouce selon la revendication 1, dans laquelle la première bande de support incurvée ne s'étend pas latéralement vers un petit doigt (136) de la main sensiblement au-delà de la gouttière palmaire.

4. Attelle de pouce selon la revendication 1, dans laquelle la troisième bande de support incurvée ne s'étend pas latéralement vers un petit doigt (136) de la main sensiblement au-delà d'une ligne médiane (138) de la main.

5. Attelle de pouce selon la revendication 1, dans laquelle la première bande de support incurvée entoure sensiblement au moins une partie proximale d'un métacarpien de pouce (106) sur la paume et/ou le talon de la main.

6. Attelle de pouce selon la revendication 1, dans laquelle la deuxième bande de support incurvée 116 entoure sensiblement au moins une partie proximale de l'articulation métacarpo-phalangienne du pouce sur la paume de la main entre une phalange de pouce (140) et un métacarpien de pouce (106).

7. Attelle de pouce selon la revendication 1, dans laquelle la troisième bande de support incurvée entoure sensiblement une partie proximale de l'articulation intercarpienne du pouce le long d'un côté arrière et/ou d'un côté d'un poignet (132) de la main.

8. Attelle de pouce selon la revendication 1, dans laquelle la quatrième bande de support incurvée entoure sensiblement au moins une partie proximale de l'articulation métacarpo-phalangienne du pouce sur la face arrière de la main entre une phalange de pouce (140) et un métacarpien de pouce (106).

9. Attelle de pouce selon la revendication 1, dans laquelle les première, deuxième, troisième et quatrième bandes de support incurvées comprennent des bandes d'un matériau élastomère ou d'un tissu, le matériau élastomère ou le tissu étant renforcé avec un matériau semi-rigide.

10. Attelle de pouce selon la revendication 1, dans laquelle aucune des première, deuxième, troisième ou quatrième bandes de support incurvées ne s'enroule autour du pouce de la paume vers la face arrière de la main.

11. Procédé de fabrication d'une attelle de pouce (100) pour immobiliser au moins partiellement un pouce (102) d'un utilisateur, le procédé comprenant les étapes consistant à :
former une première bande de support incurvée (108) configurée pour être disposée le long d'une articulation intercarpienne (112) du pouce et dans une gouttière palmaire (114) sur un talon (110) d'une main (104) ;
former une deuxième bande de support incurvée (116) couplée à la première bande de support incurvée et configurée pour s'étendre le long d'une articulation métacarpo-phalangienne (120) du pouce sur une paume (118) de la main et, ainsi, exposer une partie de la paume entre les première et deuxième bandes de support incurvées ;
former une troisième bande de support incurvée (122) configurée pour être disposée le long de l'articulation intercarpienne du pouce sur une face arrière (124) de la main ;
former une quatrième bande de support incurvée (126) couplée à la troisième bande de support incurvée et configurée pour s'étendre le long de l'articulation métacarpo-phalangienne du pouce sur la face arrière de la main et, ainsi, exposer une partie de la face arrière de la main entre les troisième et quatrième bandes de support incurvées ;
former un élément de jonction (128) couplant la première bande de support incurvée, la deuxième bande de support incurvée, la troisième bande de support incurvée et la quatrième bande de support incurvée le long d'un côté de la main défini par un métacarpien de pouce (106) de la main ; et
coupler une première sangle (134) à l'élément de jonction de telle sorte que la première sangle soit configurée pour s'enrouler autour du pouce et se coupler à au moins l'une des deuxième et quatrième bandes de support incurvées,
**caractérisé en ce que** :
au moins une de la première bande de support incurvée, de la deuxième bande de support incurvée, de la troisième bande de support incurvée et de la quatrième bande de support incurvée, présente une courbure concave, et
la courbure concave est formée pour correspondre à une courbure locale respective d'une partie de la main sur laquelle au moins une des première, deuxième, troisième et quatrième bandes de support incurvées est configurée pour venir en contact.

12. Procédé selon la revendication 11, comprenant en outre l'étape consistant à :
coupler une seconde sangle (130) à la première bande de support incurvée de telle sorte que la première sangle soit configurée pour s'enrouler autour d'un poignet (132) de la main et se coupler à la troisième bande de support incurvée.
